(19)

Europäisches
Patentamt
European
Patent Office
Office européen
des brevets

(11) **EP 4 176 874 A1**

(12) **EUROPEAN PATENT APPLICATION**

(43) Date of publication:
**10.05.2023  Bulletin 2023/19**

(21) Application number: **21207041.1**

(22) Date of filing: **09.11.2021**

(51) International Patent Classification (IPC):
*A61K 31/138* (2006.01)   *A61K 31/522* (2006.01)
*A61K 9/28* (2006.01)     *A61K 9/20* (2006.01)
*A61K 31/4439* (2006.01)   *A61K 9/16* (2006.01)

(52) Cooperative Patent Classification (CPC):
**A61K 31/4439; A61K 9/1611; A61K 9/1635;
A61K 9/1652; A61K 9/1682; A61K 9/2846;
A61K 9/286; A61K 9/2893; A61K 31/522**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Evonik Operations GmbH
45128 Essen (DE)**

(72) Inventors:
• **GUHA, Ashish
421204 Mumbai (IN)**

• **PODDAR, Aditi
421501 Ambernath (E) (IN)**
• **NOLLENBERGER, Kathrin
62495 Darmstadt (DE)**
• **JOSHI, Shraddha
400708 Thane (IN)**

(74) Representative: **Evonik Patent Association
c/o Evonik Industries AG
IP Management
Bau 1042A/PB 15
Paul-Baumann-Straße 1
45772 Marl (DE)**

(54) **PHARMACEUTICAL OR NUTRACEUTICAL COMPOSITION WITH AN ALCOHOL RESISTANT SINGLE LAYER COATING SYSTEM**

(57)    A gastric resistant pharmaceutical or nutraceutical composition, comprising a core, comprising a pharmaceutical or nutraceutical active ingredient and a single alcohol resistance conferring, gastric resistant coating layer onto the core, the coating layer comprising a mixture of 40 - 95 % by weight on the basis of the dry weight of the coating layer of an enteric core/shell polymer composition derived from an emulsion polymerisation process and of 5 - 60 % by weight on the basis of the dry weight of the coating layer of one or more salts of alginic acid, wherein the core of the core/shell polymer composition is formed by a water-insoluble copolymer and the shell of the core/shell polymer composition is formed by an anionic copolymer.

A process for producing said gastric resistant pharmaceutical or nutraceutical composition using an aqueous system in a spray process or in a fluidized bed coating equipment coupled with bottom spray, tangential spray or a top spray assembly.

EP 4 176 874 A1

**Description**

The present invention provides a gastric resistant pharmaceutical or nutraceutical composition, which is resistant to the influence of ethanol in gastric environment and thus can prevent alcohol induced dose dumping. The pharmaceutical or nutraceutical composition comprises a core, comprising a pharmaceutical or nutraceutical active ingredient and a single gastric resistant coating layer onto the core.

Pharmaceutical or nutraceutical compositions are designed to release the active ingredient in a manner of reproducible release curves. This shall result in desirable and reliable blood level profiles which shall provide an optimal therapeutic effect. If the blood level concentrations are too low, the active ingredient will not cause a sufficient therapeutic effect. If the blood level concentrations are too high, this may cause toxic effects. In both cases non optimal blood level concentrations of an active ingredient can be dangerous for the patient and shall therefore be avoided. A problem exists in that the ideal ratios assumed for the release of active ingredient during the design of a pharmaceutical or nutraceutical composition can be altered by the general living habits, thoughtlessness or by addictive behaviour of the patients with respect to the use of ethanol or ethanol-containing drinks. In these cases, the pharmaceutical or nutraceutical form which is actually designed for an exclusively aqueous medium is additionally exposed to an ethanol containing medium of greater or lesser strength. Since health authorities like for instance the US Food and Drug Administration (FDA) focus more and more on the ethanol problem, ethanol resistance may be an important registration requirement in the near future.

Since not all patients are aware of the risk of simultaneous taking of a controlled release pharmaceutical or nutraceutical form and ethanol-containing drinks or do not follow or are not able to follow appropriate warnings, advice or recommendations, there is a demand for controlled release pharmaceutical or nutraceutical compositions, especially for gastric resistant pharmaceutical or nutraceutical compositions, such that their mode of action is affected as little as possible by the presence of ethanol.

Conventional gastric resistant pharmaceutical or nutraceutical compositions if coated or uncoated are usually not resistant to alcohol at all. Especially there is a problem for gastric resistant or enteric formulated compositions. These kinds of formulations are usually coated with a gastric resistant coating layer (enteric coating layer) onto the core which has the function that the release of the pharmaceutical or nutraceutical active ingredient in the stomach, respectively at pH 1.2 for 2 hours according to USP, shall not exceed 10 %, preferably less than 5 %. This function ensures that acid-sensitive pharmaceutical or nutraceutical active ingredients are protected against inactivation and that pharmaceutical or nutraceutical active ingredients which may be irritate the stomach mucosa are not set free in too high amounts. On the other hand, in many cases the release of the pharmaceutical or nutraceutical active ingredient in the intestine, respectively at pH 6.8 for one hour or less according to the USP method, is designed to exceed at least 70, 75 % or more. The presence of ethanol in concentrations of 5, 10, 20 or 40 % (volume/volume) in the gastric fluid usually leads to an increase to the release rates in the stomach. Due to distribution effect the effect of ingested ethanol is in the intestine not of that importance as in the stomach. Thus, an effective protection against the influence of ethanol should prevent such an undesired increase of pharmaceutical or nutraceutical active ingredient in the stomach in the first place. Furthermore, it may be desired that protection against the influence of ethanol shall at least not influence the comparably fast release rates at pH 6.8 in media without ethanol.

Therefore, lots of efforts have been made in the past to provide pharmaceutical or nutraceutical compositions with a gastric resistant release profile that are also resistant against the influence of ethanol at pH 1.2.

WO 2012171575 A1 and WO 2012171576 A1 are concerned with a coating composition for enteric release profile suitable for coating of a pharmaceutical or nutraceutical dosage form. The coating composition is comprising at least 20% by weight of an enteric core/shell polymer composition derived from an emulsion polymerization process, wherein either the core is formed by a water-insoluble, not cross linked polymer or copolymer and the shell is formed an anionic polymer or copolymer or vice versa. Alcohol resistance of such coatings has also been demonstrated and claimed against 20% v/v ethanol in 0.1N HCl. Experimentally, it has been found that these coatings are not resistant to 40% v/v ethanol content in 0.1N HCl. However, regulatory authorities require that such resistance is demonstrated in various products.

For the purpose of alcohol resistance, US 20120093926 A1 and WO 2012022498 A1 propose a gastric resistant pharmaceutical or nutraceutical composition, comprising a core, comprising a pharmaceutical or nutraceutical active ingredient and a gastric resistant coating layer onto the core, wherein the release of the pharmaceutical or nutraceutical active ingredient is not more than 15% under in-vitro conditions at pH 1.2 for 2 hours in medium according to USP with and without the addition of 40% (v/v) ethanol, wherein the gastric resistant coating layer comprises 10 to 100% by weight of one or more salts of alginic acid with a viscosity of 30 to 720 cp of a 1% aqueous solution. The documents also disclose the requirement of curing to address stability problems in case of multiple polymers. This stability problem has been found to be more relevant in case of combinations of EUDRAGIT® NM 30 D, a commercially available methyl methacrylate - ethyl acrylate copolymer (i.e. poly(ethyl acrylate-co-methyl methacrylate) 2:1)) dispersion, and sodium alginate. Moreover, it is required that such coatings provide alcohol resistance at least for the period of 2 hours.

WO 2020249505 A1 discloses a powder composition, comprising 50 to 95% by weight of a copolymer mixture A of a copolymer 1, comprising 5 to 60% by weight of polymerized units of methacrylic acid and 95 to 40% by weight $C_1$- to $C_4$- alkyl esters of (meth)acrylic acid, and a copolymer 2, comprising more than 95 and up to 100% by weight of polymerized units of $C_1$- to $C_4$-alkylesters of (meth)acrylic acid, and 50 to 5% by weight of a water-soluble cellulose B. The powder composition originates from the co-processing of the copolymer mixture A and the water-soluble cellulose B. The powder composition originates from the co-processing of the copolymer mixture A and water-soluble cellulose B by a drying process of an aqueous dispersion, such as spray drying or freeze drying. Further processing leads to a compressed dosage form. This document focuses on the properties of the core shell polymer wherein curing free matrix tableting systems are mentioned and does not mention coating systems.

WO 2020225122 A1 is concerned with a pellet comprising a core, comprising one or more biologically active ingredients, and a coating layer onto the core, wherein the coating layer is comprising a mixture of a first polymer, which is a core-shell polymer, comprising 50 to 90 % by weight of a core, comprising polymerized units of 60 to 85 % by weight of ethyl acrylate and 20 to 40 % by weight of methyl methacrylate, and 10 to 50 % by weight of a shell, comprising polymerized units of 40 to 60 % by weight ethyl acrylate and 40 to 60 % by weight methacrylic acid, and a second polymer, comprising polymerized units of 40 to 60 % by weight of methacrylic acid and 60 to 40 % by weight of ethyl acrylate or methyl methacrylate, wherein the ratio of the first polymer to the second polymer is from about 1:0.1 to 1:10. Also disclosed is a Multi-Unit Pellet System (MUPS), preferably a compressed tablet, comprising a multitude of the pellets. This document also describes the functionality of core/shell polymer alone and in combination with other methacrylate polymers for the formulation of MUPS, but the property of alcohol resistance is not mentioned.

WO 2014032741 A1 refers to a pharmaceutical or nutraceutical composition, comprising a) a core, comprising a pharmaceutical or a nutraceutical active ingredient and b) an inner coating layer comprising at least 30% by weight of one or more salts of alginic acid and c) an outer coating layer comprising at least 30% by weight of one or more polymers or copolymers comprising anionic side groups. In the comparative examples, it is disclosed that a combination of methacrylate polymer and sodium alginate, in a monolayer fails to give alcohol resistance even at a polymeric build-up of 100%. Therefore, a bilayer system has been proposed. Such bilayer coating can lead to increased process time and compromised process efficiency.

WO 2014032742 A1 refers to a pharmaceutical or nutraceutical composition, comprising a) a core, comprising a pharmaceutical or a nutraceutical active ingredient and b) an inner coating layer comprising one or more salts of alginic acid and c) an outer coating layer comprising one or more water-insoluble polymers or copolymers, wherein the ratio of the amount of the one or more salts of alginic acid in the inner coating layer is at least 2.5 : 1 by weight to the amount of the one or more water-insoluble polymers or copolymers in outer coating layer. A monolayer coating system with mix of methacrylate polymer and sodium alginate for alcohol resistance is not disclosed in the document.

WO 2015121189 A1 refers to a pharmaceutical or nutraceutical composition, comprising (a) a core, comprising a pharmaceutical or a nutraceutical active ingredient, b) an inner coating layer comprising one or more salts of alginic acid and c) an outer coating layer comprising one or more water-insoluble polymers or copolymers and one or more water-soluble polymers, selected from the group of water soluble celluloses, polyvinyl pyrrolidone or polyethylene glycols or any combination thereof, wherein the ratio by weight of the amount of the one or more salts of alginic acid in the inner coating layer to the amount of the one or more water-insoluble polymers or copolymers in outer coating layer is from 0.6 to less than 2.5:1. A monolayer coating system with a mix of methacrylate polymer and sodium alginate for alcohol resistance is not disclosed in the document.

A delayed release formulation usually contains a film coat that prevent the drug release in gastric environment. The integrity of this film can come under question if the medication is concomitantly ingested with ethanol. Alcohol causes a disruption in the drug release mechanism leading to the entire dose being released at once due to ingestion of ethanol. This becomes a more serious problem if the formulation contains a drug with narrow therapeutic index, leading to risk in patient safety.

The alcohol resistance can be achieved using methacrylate polymers in combination with other excipients, in a single layer, but this approach suffers from multiple limitations like

  1. Neutralization step for preparation of the coating dispersion
  2. Curing requirement leading to stability problems.
  3. Processing problems like spray drying, reduced coating efficiency.

A bi-layer coating approach has also been attempted to achieve alcohol resistance, but it has problems like

  1. Two-layer requirement.
  2. Long processing time.
  3. Complex coating calculations
  4. Compromised process efficiency.

Thus, there is still a need to improve the coating composition and process for delayed release formulations intended to have alcohol resistance or resists alcohol dose dumping.

Therefore, the present invention concerns a gastric resistant pharmaceutical or nutraceutical composition, comprising a core, comprising a pharmaceutical or nutraceutical active ingredient and a single alcohol resistance conferring, gastric resistant coating layer onto the core, the coating layer comprising a mixture of 40 - 95 %, preferably 50% - 95%, more preferably 60% - 90%, by weight on the basis of the dry weight of the coating layer of an enteric core/shell polymer composition derived from an emulsion polymerisation process and of 5 - 60 %, preferably 5% - 50% and more preferably 10% - 40%, by weight on the basis of the dry weight of the coating layer of one or more salts of alginic acid, wherein the core of the core/shell polymer composition is formed by a water-insoluble copolymer and the shell of the core/shell polymer composition is formed by an anionic copolymer.

Optionally, the core of the gastric resistant pharmaceutical or nutraceutical composition according to the present invention may comprise further coating layers having functionalities, other than conferring alcohol and/or gastric resistance, besides the single alcohol resistance conferring, gastric resistant coating layer onto the core. In particular, the composition according to the present invention may further comprise one or more additional layers in order to achieve one or more of the functionalities, selected from modified release, odour masking, taste masking, improvement in optics, stabilization and moisture protection.

Enteric core/shell polymer compositions derived from an emulsion polymerisation process are disclosed in WO 2012171575 A1.

The anionic copolymer of the enteric core/shell polymer composition that is derived from an emulsion polymerisation process is preferably an anionic (meth)acrylate copolymer comprising polymerized units of 40 to 60% by weight methacrylic acid, and 60 to 40% by weight ethyl acrylate, preferably 50 % by weight methacrylic acid and 50 % by weight ethyl acrylate.

The water-insoluble copolymer of the enteric core/shell polymer composition may comprise (meth)acrylate copolymers, preferably comprising polymerized units of 20 to 40 % by weight methyl (meth)acrylate and of 60 to 80 % by weight ethyl acrylate, preferably polymerized units of 30 % by weight methyl (meth)acrylate and of 70 % by weight ethyl acrylate.

The one or more salts of alginic acid in the coating layer ideally have a viscosity of 10 to 720 mPa·s in a 1 % aqueous solution. The salt of alginic acid is preferably sodium alginate.

The core comprised in the gastric resistant pharmaceutical or nutraceutical composition according to the present invention may comprise or may contain a neutral carrier pellet, for instance a sugar sphere or non-pareilles, on top of which the active ingredient is bound in a binder, such as lactose or polyvinylpyrrolidone. The core may alternatively comprise a pellet in the form of a polymeric matrix in which the active ingredient is bound. The core may comprise an uncoated pellet consisting of a crystallized active ingredient. The core may be as well a tablet, a mini tablet or a capsule.

The single alcohol resistance conferring, gastric resistant coating layer onto the core has the function that the release of the pharmaceutical or nutraceutical active ingredient is not more than 10 % under in-vitro conditions at pH 1.2 for 2 hours in medium according to USP (USP = United States Pharmacopoeia) with and without the addition of 40 % (v/v) ethanol. The USP which may be preferably used is USP35 / NF 30 (NF = National Formulary), apparatus II, paddle method, or basket method. Acceptance criteria for dissolution profiles as described in USP 35 general chapter <711> 'Dissolution' can be used.

The release of the pharmaceutical or nutraceutical active ingredient may be at least 70, preferably at least 75 %, most preferably at least 80 % under in-vitro conditions at pH 6.8 or other suitable pH conditions, within the time as specified in individual monographs or as per the formulation specific needs, with and without the addition of 40 % (v/v) ethanol.

The gastric resistant pharmaceutical or nutraceutical composition according to the present invention is comprising a core, comprising a pharmaceutical or nutraceutical active ingredient. The pharmaceutical or nutraceutical active ingredient may be inactivated under the influence of gastric fluids at pH 1.2 or a pharmaceutical or nutraceutical active ingredient which may irritate the stomach mucosa when set free in the stomach.

Nutraceutical active ingredients can be defined as extracts of foods claimed to have medical effects on human health. The nutraceutical ingredients or dietary supplements that may be comprised in the core of the gastric resistant nutraceutical composition according to the present invention that are of particular relevance and advantage are oils, enzymes, prebiotics, probiotics, vitamins, antioxidants, dietary fibres, and its derivatives.

Nutraceuticals and dietary supplements of interest described above can be contained in a medical format such as capsule, tablet, pellet, granules or powder in a prescribed dose. Examples for nutraceuticals that are of particular relevance here are; fish oils, bacteria and spores such as Lactobacillus casei, bifidobacterium longum and *Bacillus coagulans,* Pancreatic enzymes, Hydroxytyrosol, Resveratrol, Cobalamin, Pantothenic acid, Isoflavonoids and polysaccharides.

Therapeutical and chemical classes of pharmaceutical active ingredients that may be comprised in the core of the gastric resistant pharmaceutical composition according to the present invention that are of particular relevance and advantage are; antimicrobials, hormones, enzymes, enzyme inhibitors, receptor agonists & antagonists, oral vaccines, proteins, peptides and its combinations.

Examples of pharmaceutical active ingredients, which are acid-lablile, irritating or need controlled release, may be: Dexlansoprazole, Lansoprazole, Omeprazole, Minoprazole, Pantoprazole, Rabeprazole, Erythromycin, Ampicillin, Doxycycline, Fluoxetin, Ketoprofen, Oxymorphone Hydrochloride, Tramadol, Hydromorphone Hydrochloride, Sulfazsalazine, Mesalamine, Didanosine, Mycophenolate mofetil, Heparin, Human interleukin-10, Human Growth Hormone, IgG antibodies, including their salts, derivatives, polymorphs, isomorphs or any kinds of mixtures or combinations thereof.

The gastric resistant pharmaceutical or nutraceutical composition may be a coated tablet, a coated minitablet, a coated pellet, a coated granule, a sachet, a coated hard-shell capsule, coated soft-shell capsule, filled with coated pellets or with powder or with granules, or a coated capsule, filled with oils, coated pellets or with powder or with granules. The term coated tablet includes pellet-containing tablets or compressed tablets and is well known to a skilled person. Such a tablet may have a size of around 5 to 25 mm for instance. Usually, defined pluralities of small active ingredient containing pellets are compressed therein together with binding excipients to give the well-known tablet form. After oral ingestion and contact with the body fluid the tablet form is disrupted and the pellets are set free. The compressed tablet combines the advantage of the single dose form for ingestion with the advantages of a multiple forms, for instance the dosage accuracy.

The term coated minitablet is well known to the skilled person. A minitablet is smaller than the traditional tablet and may have a size of around 1 to 5 mm. The minitablet is, like a pellet, a single dosage form to be used in multiple dosages. In comparison to pellets, which may be in the same size, minitablets usually have the advantage of having more regular surfaces which can be coated more accurately and more uniformly. Minitablets may be provided enclosed in capsules, such as gelatine capsules. Such capsules disrupt after oral ingestion and contact with the gastric or intestinal fluids and the minitablets are set free. Another application of minitablets is the individual fine adjustment of the active ingredient dosage. In this case the patient may ingest a defined number of minitablets directly which matches to the severe of the decease to cure but also to his individual body weight. A minitablet is different from pellet-containing compressed tablet as discussed above.

The terms Pellets or granules are well known to the skilled person and may be used as core for coating or in compressed tablets or filled in capsules after coating. Such pellets or granules may have a size in the range of 50 to 1000 mm (average diameter), Such pellets or granules can be produced using processes such as high and low shear granulation, extrusion and spheronization, prilling, active layering, compaction, agglomeration and fluidised bed processes.

The term sachet is well known to the skilled person. It refers to small sealed package which contains the active ingredient often in pellet containing liquid form or also in dry pellet or powder form. The sachet itself is only the package form is not intended to be ingested. The content of the sachet may be dissolved in water or as an advantageous feature may be soaked or ingested directly without further liquid. The latter is advantageous feature for the patient when the dosage form shall be ingested in a situation where no water is available. The sachet is an alternative dosage form to tablets, minitablets or capsules.

Coated pellets may be filled in a capsule, for instance gelatine or HPMC capsule. A capsule containing pellets may also be coated with the enteric coating layer according to the invention.

The present invention further concerns a coating composition that is suitable for preparing the gastric resistant pharmaceutical or nutraceutical composition according to the present invention and comprises a mixture of 40 - 95 %, preferably 50% - 95%, more preferably 60% - 90%, by weight on the basis of the dry weight of the coating layer of an enteric core/shell polymer composition derived from an emulsion polymerisation process, wherein the core of the core/shell polymer composition is formed by a water-insoluble polymer or copolymer and the shell of the core/shell polymer composition is formed by an anionic copolymer and of 5 - 60 %, preferably 5% - 50% and more preferably 10% - 40%, by weight on the basis of the dry weight of the coating layer of one or more salts of alginic acid.

The enteric core/shell polymer composition may be present in the coating composition in form of the solid phase of an aqueous dispersion with a solid content from 1 to 60 % by weight. This means that the aqueous polymer dispersion, that is used to prepare the coating formulation, may contain 1 to 70 % by weight of the coating composition as solid phase and 30 to 99 % by weight as aqueous phase.

The enteric core/shell polymer composition comprised in the coating composition that can be used for the preparation of the gastric resistant pharmaceutical or nutraceutical composition according to the present invention is derived from an emulsion polymerisation process (cf. WO2012171575 A1).

In a typical emulsion polymerisation process first a core in the form of core particles is formed by polymerisation of the monomers for polymer or the copolymer of the core. Subsequently the monomers for polymer or the copolymer of the shell are polymerized in the same reaction mixture to give a shell around on the surface of the core particles.

It may as well possible to start the emulsion polymerisation process first by the addition of readily polymerized polymer particles such as cellulose particles or starch particles to the polymerisation mixture. Subsequently the monomers for polymer or the copolymer of the shell are polymerized in this reaction mixture to give a shell around on the surface of readily polymerized polymer core particles.

In the emulsion polymerization process, the operation may advantageously be carried out by the monomer emulsion feed process or the monomer feed process, respectively. For this, water is heated to the reaction temperature in the polymerization reactor. Surfactants and/or initiators may be added at this stage. Then - depending on the mode of operation - the monomer, a monomer mixture or an emulsion of either are fed to the reactor. This dosed liquid may contain initiators and/or surfactants or the initiator and/or the surfactant may be dosed parallel.

Alternatively, all monomers for the core can be charged into the reactor, before adding the initiator. This method is often referred to as batch process.

It is also possible to do a combination of both processes, by polymerizing a part of the monomers in the manner of a batch process and feeding the other part afterwards. As known to the expert in the field, the type of process and mode of operation can be chosen, to achieve the desired particle size, sufficient dispersion stability, a stable production process and so on.

The average particle size of the polymer particles produced in the emulsion polymerization may range from 10 to 1000, 20 to 500 or 50 to 250 nm. The average particle size of the polymer particles may be determined by methods well known to a skilled person, for instance by the method of laser diffraction. The particle size may be determined by laser diffraction, using a Mastersizer® 2000 (Malvern). The values can be indicated as particle radius rMS [nm], which is half of the median of the volume-based particle size distribution d(v,50).

Emulsifiers which may be used are especially anionic and non-ionic surfactants. The amount of emulsifier used is generally not more than 5% by weight, based on the polymer. Typical surfactants are for example alkyl sulfates (e.g. sodium dodecyl sulfate), alkyl ether sulfates, dioctyl sodium sulfosuccinate, polysorbates (e.g. polyoxyethylene (20) sorbitan monooleate), nonylphenol ethoxylates (nonoxynol-9) and others.

Beside those initiators conventionally used in emulsion polymerization (e.g. per-compounds, such as ammonium peroxodisulfate (APS), redox systems, such as sodium disulphite-APS-iron can be applied. Water-soluble azo initiators may also be applied and/or a mixture of initiators can be used. The amount of initiator is usually between 0.005 to 0.5% by weight, based on the monomer weight.

The polymerization temperature depends on the initiators within certain limits. For example, if APS is used it is advantageous to operate in the range from 60 to 90° C; if redox systems are used it is also possible to polymerize at lower temperatures, for example at 30° C.

Preferably, the core/shell polymer composition comprised in the coating composition that is suitable for preparing the gastric resistant pharmaceutical or nutraceutical composition according to the present invention comprises 30 % by weight of the copolymers of the core and 70 weight of the copolymers of the shell.

The anionic copolymer comprised in the shell of the enteric core/shell polymer composition comprised in the coating composition that is suitable for preparing the gastric resistant pharmaceutical or nutraceutical composition according to the present invention is preferably an anionic (meth)acrylate copolymer comprising polymerized units of 40 to 60% by weight methacrylic acid, and 60 to 40% by weight ethyl acrylate, preferably 50 % by weight methacrylic acid and 50 % by weight ethyl acrylate.

Examples for such commercially available anionic copolymers are e.g. EUDRAGIT® L or EUDRAGIT® L100 55 types. EUDRAGIT® L is a copolymer of 50% by weight methyl methacrylate and 50% by weight methacrylic acid. The pH of the start of the specific active ingredient release in intestinal juice or simulated intestinal fluid can be stated to be pH 6.0. EUDRAGIT® L 100-55 is a copolymer of 50% by weight ethyl acrylate and 50% by weight methacrylic acid. EUDRAGIT® L30 D-55 is a dispersion comprising 30% by weight EUDRAGIT® L 100-55. The pH of the start of the specific active ingredient release in intestinal juice or simulated intestinal fluid can be stated to be pH 5.5.

An anionic (meth)acrylate copolymer may be produced by radical polymerisation of the monomers in the presence of polymerisation initiators. Molecular weight regulators may be added. The preferred polymerisation method is emulsion polymerisation (cf. e.g. WO 2012171575 A1).

The water-insoluble copolymer comprised in the core of the enteric core/shell polymer composition comprised in the coating composition that is suitable for preparing the gastric resistant pharmaceutical or nutraceutical composition according to the present invention may comprise (meth)acrylate copolymers, preferably comprising polymerized units of 20 to 40 % by weight methyl (meth)acrylate and of 60 to 80 % by weight ethyl acrylate, preferably polymerized units of 30 % by weight methyl (meth)acrylate and of 70 % by weight ethyl acrylate.

Suitable examples of such commercially available water-insoluble copolymers are EUDRAGIT® NE 30D or EUDRAGIT® NM 30D type (cf. WO 2012171575 A1). EUDRAGIT® NE 30D and Eudragit® NM 30D are dispersions containing 30 % by weight of copolymers composed of free-radically polymerized units of 70% by weight of ethyl acrylate and 30% by weight of methyl methacrylate. Preference is given to neutral or essentially neutral methyl acrylate copolymers which, according to WO0168767 A1, have been prepared as dispersions using 1 - 10% by weight of a non-ionic emulsifier having an HLB value of 15.2 to 17.3. The latter offer the advantage that there is no phase separation with formation of crystal structures by the emulsifier (Eudragit® NM 30D type).

The one or more salts of alginic acid in the coating composition that is suitable for preparing the gastric resistant pharmaceutical or nutraceutical composition according to the present invention ideally have a viscosity of 10 to 720 mPa·s in a 1 % aqueous solution.

The methodology of determination of the viscosity of a polymer solution, for instance a solution of a salt of alginic acid, is well known to the skilled person. The viscosity is preferably determined according to European Pharmacopeia 7th edition, general chapter 2, methods of analysis, 2.2.8 and 2.2.10, page 27ff. The test is performed using a spindle viscometer. The viscosity of a 1% alginate solution may be determined by adding 3 g product to 250 ml of distilled water in a beaker while stirring at 800 rpm using overhead stirrer. Then additional 47 ml water was added with rinsing the walls of the beaker. After stirring for 2 hours and getting a complete solution, the viscosity is measured using a LV model of the Brookfield viscometer at 25°C (77°F) at 60 rpm with no. 2 spindle for samples with a viscosity of more than 10 Pa s and at 60 rpm with no. 1 spindle for samples with viscosity less than 10 Pa s. Since the weight of water is almost exactly 1g/ml even at 25 °C "weight/weight" is regarded as equal or identical to "weight/volume" in the sense of the invention. Theoretically possible marginal differences are regarded as insignificant.

The salts of alginic acid in the coating composition that is suitable for preparing the gastric resistant pharmaceutical or nutraceutical composition according to the present invention may be selected from sodium alginate, potassium alginate, magnesium alginate, lithium alginate or ammonium alginate or mixtures thereof. The salt of alginic acid is preferably sodium alginate.

The present invention also concerns a process for producing the gastric resistant pharmaceutical or nutraceutical composition according to the present invention by forming the core comprising a pharmaceutical or nutraceutical active ingredient using one or more processes known in the prior art and by applying the coating composition according to the present invention in the form of an aqueous dispersion in a coating pan using a spray process or in a fluidized bed coating equipment which may have bottom spray, tangential spray or a top spray assembly. Bottom spray process may involve the coating of fluidized particles with coating solution/ dispersion being sprayed in the direction of air flow. Tangential spray involves a rotor disc which sets the particles in a spiral motion, the coating solution is sprayed tangentially to the rotating disc and in the direction of the particle movement. This technique has the advantage of spraying liquids with faster high solid deposition in the form of powder. Top spray coating process involves the coating of fluidized particles by spraying the coating liquid against the air flow.

**Experimental Part**

**A. Core details:**

    **1. Theophylline pellets (18#-20#) commercially procured.**
    **2. Metoprolol pellets (18#-20#) prepared in lab by drug loading on non pareil seeds**

**Preparation of Metoprolol Succinate pellets by drug layering**

**Core used :** Non pareil seeds (size 707-841 microns)
**Quantity taken** : 600.0 gm

| Formula: Ingredients | Manufacturer | Solid content (g) | Qty. batch (g) |
|---|---|---|---|
| Metoprolol succinate | Polydrug lab Pvt Ltd | 600.0 | 600.0 |
| Polyvinylpyrroli done (Kollidon 30) | BASF | 150.0 | 150.0 |
| Water | | | 1593.7 |
| Total | | 750.0 | 2343.7 |

Total solid content: 32% w/w

**Procedure for drug layering suspension preparation:**

    1. Polyvinylpyrrolidone was accurately weighed and dissolved in water using an overhead stirrer.

2. Metoprolol succinate was added to suspension of step 1 and stirring was continued for 60 minutes.

3. The final prepared suspension was passed through a sieve of 250 microns (60#).

4. This suspension was further sprayed onto pellets in fluid bed processor.

5. After completion of spraying pellets were dried in fluid bed processor till the LOD was less than 2% w/w

**Equipment and in process coating parameters:**

Machine parameters: GPCG 3.1

| Parameters | Unit | Value |
|---|---|---|
| **Equipment setup** | | |
| Nozzle bore | Mm | 0.8 |
| Internal silicone tube diameter | Mm | 5 |
| **Process parameter setup** | | |
| Atomizing air pressure | Bar | 1.0 - 1.1 |
| Filter shaking pause | Sec | 150 |
| Filter shaking | Sec | 05 |
| Air flow | $m^3/h$ | 128 - 135 |
| **Process data** | | |
| Inlet Temperature | °C | 60 - 63 |
| Product Temperature | °C | 44 - 52 |
| Spray rate | g/min | 5 - 9.7 |

**3. Caffeine pellets preparation by Extrusion Spheronization:**

**Extrusion spheronisation method:**

| Ingredients | Manufacturer | Solid content (g) | Qty. batch (g) |
|---|---|---|---|
| Caffeine anhydrous | Aarti Drugs | 200.0 | 200.0 |
| Microcrystalline cellulose (Avicel CL 611) | FMC Biopolymers | 100.0 | 100.0 |
| Microcrystalline cellulose (Avicel 101) | FMC Biopolymers | 200.0 | 200.0 |
| Water | | | 580.0 |
| Total | | 500.0 | 1080.0 |

**Procedure:**

1. Caffeine was sifted through 60 mesh sieve.

2. Other ingredients were sifted through 40 mesh sieve.

3. All the materials were blended together in polybag for 5 minutes.

4. The blend was mixed in planetary mixer for 5 minutes at slow speed.

5. Purified water was added to the blend for 5 minutes at slow speed and then mixing was continued for another 10 minutes at fast speed.

6. The mixture of step 5 was taken for extrusion in two lots using the following parameters:

**Extrusion parameters:**

Extruder type: Cone Type of screw rotation: single

Extruder screw: Double Screw speed : 50 RPM

Extrusion pressure: 1.2 - 1.4 bar Screen diameter: 1 mm

Extrusion temperature: 20-25°C Feed rate: 20-25 g/min

Extrusion time: 30 min

The extrudes were spheronised in 3 equal lots using the following parameters:

**Spheronisation parameters:**

Spheronizer plate type: 2 mm
Spheronizer speed: 1750 for 2 minutes, 1000 for 20 seconds, 700 for 2 minutes and finally 500 for 2 minutes
Spheronization time: 6 min
LOD: Less than 2% after drying

4. Garlic soft gelatin capsules commercially procured.
5. Lansoprazole was commercially procured.

**B. Comparative examples**

**(1) Formulae for the comparative examples**

| Examples | C1 DS (g) | C1 Qty (g) | C2 DS (g) | C2 Qty (g) | C3 DS (g) | C3 Qty (g) | C4 DS (g) | C4 Qty (g) | C5 DS (g) | C5 Qty (g) | C6 DS (g) | C6 Qty (g) | C7 DS (g) | C7 Qty (g) | C8 DS (g) | C8 Qty (g) |
|---|---|---|---|---|---|---|---|---|---|---|---|---|---|---|---|---|
| Formulation details | Theophylline pellets (18#-20#) coating with EUDRAGIT® FL 30 D-55 | | Metoprolol pellets (18#-20#) coated with mix of EUDRAGIT® NM 30D and sodium alginate | | | | | | Caffeine pellets (16#-40#) coated with mix of EUDRAGIT® L 30D-55 and sodium alginate | | | | Bilayer coating on Caffeine pellets of inner layer sodium alginate and outer layer EUDRAGIT® L 30D-55 | | Theophylline pellets (18#-20#) coating with mix of EUDRAGIT® NM 30D+ EUDRAGIT® L 30 D-55 and sodium alginate | |
| Batch size (g) | 400 | | 500 | | 500 | | 500 | | 600 | | 600 | | 550 | | 400 | |
| Ratio of polymer in coating | NA | | NM:Sod alg = 70:30 | | NM:Sod alg = 80:20 | | NM:Sod alg = 90:10 | | L: Sod Alg = 40:60 | | L: Sod Alg = 66:33 | | NA | | Physical mix: sodium alginate = 85:15 | |
| Polymeric coat build up (% w/w) w.r.t core | 70% | | 30% | | 50% | | 60% | | 140 % | | 125% | | 30% (inner layer) + 40% (outer layer) | | 70% | |
| EUDRAGIT® FL 30 D-55 | 280.0 | 933.3 | - | - | - | - | - | - | - | - | - | - | - | - | - | - |
| EUDRAGIT® NM 30 D | - | - | 140.0 | 466.6 | 200.0 | 666.6 | 225.0 | 750.0 | - | - | - | - | - | - | 178.50 | 595.0 |
| EUDRAGIT® L 30 D-55 | - | - | - | - | - | - | - | - | 336.0 | 1119.8 | 500.0 | 1666.6 | 240.0 | 800.0 | 59.5 | 198.3 |
| Sodium alginate | - | - | 60.0 | 60.0 | 50.0 | 50.0 | 25.0 | 25.0 | 504.0 | 504.0 | 250.0 | 250.0 | 165.0* | 165.0* | 42.0 | 42.0 |
| Triethyl citrate | - | - | - | - | - | - | - | - | - | - | 25.0 | 25.0 | 24.0 | 24.0 | - | - |
| Talc | 140.0 | 140.0 | 100.0 | 100.0 | 125.0 | 125.0 | 125.0 | 125.0 | 420.0 | 420.0 | 375.0 | 375.0 | 202.5 | 202.5 | 140.0 | 140.0 |

| | DS (g) | Qty (g) | DS (g) | Qty (g) | DS (g) | Qty (g) | DS (g) | Qty (g) | DS (g) | Qty (g) | DS (g) | Qty (g) | DS (g) | Qty (g) | DS (g) | Qty (g) |
|---|---|---|---|---|---|---|---|---|---|---|---|---|---|---|---|---|
| Water | | 1726. 5 | | 5373. 3 | | 5408. 3 | | 4457. 1 | | 29456. 1 | | 26433. 3 | | 5940: inner layer, 1616: outer layer | | 1824.6 |
| Solid content | 15 % w/w | | 5% w/w | | 6% w/w | | 7% w/w | | 4% w/w | | 4% w/w | | 4% w/w for inner layer, 15% w/w for outer layer | | 15% w/w | |
| *: First Layer | | | | | | | | | | | | | | | | |

**C. Process details for experiment C1**

**(1) Preparation of coating solution for pellets coating: C1**

I. To a part of water, talc was added and homogenized for 15 - 20 min.
II. Some part of water was used to rinse the homogenizer and added to Step I.
III. Dispersion of Step II added to EUDRAGIT® FL 30 D-55 under stirring.
IV. Stirring was continued till 10 min.
V. The coating dispersion was passed through 40 # ASTM sieve (425 μm)

**(2) Preparation of coating solution for pellets coating: C2 - C4**

I. To a part of water talc was homogenized for about for 15 - 20 min.
II. Some part of water was used to rinse the homogenizer and added to Step I.
III. Sodium alginate was dissolved in water under stirring for 30 min.
IV. To step III EUDRAGIT® NM 30 D was added under stirring.
V. The dispersion of Step IV was added to Step II, slowly under stirring.
VI. The final coating solution was passed through 40 # ASTM sieve (425 μm).

**(3) Preparation of coating solution for pellets coating: C5 - C6**

I. Sodium alginate was weighed and kept under stirring with water for 60 minutes on an overhead stirrer.
II. Talc was homogenized with remaining amount of water for 30 minutes.
III. Homogenized talc suspension was added to Alginate solution and stirring was continued for further 30 min.
IV. EUDRAGIT® L30 D 55 was added to alginate solution under stirring for 10 minutes.
V. The final coating solution was passed through 40 # ASTM sieve (425 μm).

**(4) Preparation of coating solution for pellets coating: C7**

**First layer coating solution preparation:**

I. Dissolve sodium alginate in 85% water to prepare a solution using an overhead stirrer for 1 hr.
II. Homogenize talc in remaining amount of water for 30 minutes.
III. Add talc suspension to alginate solution and stir for further 30 minutes.
IV. Filter final coating suspension through 40 # ASTM sieve (425 μm).

**Second layer coating solution preparation:**

I. Talc and triethyl citrate was homogenized in water for 20 minutes.
II. Homogenized talc and triethyl citrate suspension was added to EUDRAGIT® L30 D 55 dispersion under stirring using an over head stirrer and stirring was continued for further 20 mins.
III. The final prepared suspension was passed through a sieve of 40 # ASTM sieve (425 μm).
IV. This suspension was further sprayed onto pellets in fluid bed processor.

**(5) Preparation of coating solution for pellets coating: C8**

I. To a part of water talc was homogenized for about for 15 - 20 min.

II. Some part of water was used to rinse the homogenizer and added to Step I.

III. EUDRAGIT® L 30 D-55 was neutralized to pH 5 by adding 0.1 N NaOH.

IV. EUDRAGIT® NM 30 D was added to step III under stirring slowly.

V. Sodium alginate was dissolved in water under stirring for 30 min.

VI. To step V, step IV was added under stirring.

VII. The dispersion of Step VI was added to Step II, slowly under stirring.

VIII. The final coating solution was passed through 40 # ASTM sieve (425 μm)

**(6) Coating of pellets**

The general process parameters for pellet coating are enlisted below:

| Experiment number | | C1 | C2 - C4 | C5 | C6 | C7 | | C8 |
|---|---|---|---|---|---|---|---|---|
| Parameters | Unit | Value | | | | | | |
| **Equipment setup** | | | | | | | | |
| Nozzle bore | mm | 0.8 | 0.8 | 0.8 | 0.8 | 0.8 | | 0.8 |
| Internal silicone tube diameter | mm | 2.5 | 2.5 | 5.0 | 5.0 | 2.5 | | 2.5 |
| **Process parameter setup** | | | | | | | | |
| Atomizing air pressure | bar | 1.0 | 1.0 - 1.3 | 1.4 - 1.5 | 1.4 - 1.5 | 1.4 - 1.5 | 1.0 | 1.0 |
| Filter shaking Interval | sec | 60 | 100 - 250 | 300 | 300 | 100 | 50 | 60 |
| Filter shaking | sec | 05 | 05 | 05 | 05 | 04 | 04 | 05 |
| Air flow | m³/h | 50 - 60 | 50 - 90 | 140-175 | 130-175 | 75 - 90 | 80 - 90 | 50 - 60 |
| **Process data** | | | | | | | | |
| Inlet Temperature | °C | 30-31 | 35 - 40 | 68 - 73 | 68 - 73 | 66 - 81 | 40 - 44 | 30-31 |
| Product Temperature | °C | 25 - 28 | 28 - 30 | 47 - 52 | 44 0 50 | 50 - 54 | 28 - 30 | 25 - 27 |
| Spray rate | g/min | 2-6 | 4-9 | 2-21 | 2-21 | 6-15 | 5 -13 | 2-5 |
| Curing done | | No | Yes | No | No | No | No | No |

**Coated formulations were analyzed for drug release** in 0.1N HCl for 2 hours with and without presence of 40% v/v ethanol in it. Additionally release in pH 6.8 phosphate buffer after preexposure to ethanolic or non-ethanolic acidic media was also carried out. Effect of curing on drug release in pH 6.8 (after preexposure to 0.1 N HCl for 2hours) was observed for all comparative examples and storage stability on drug release in pH 6.8 (after preexposure to 0.1 N HCl for 2hours) was tested for selected examples C7 and C8. For all the dissolution/ drug release studies USP II apparatus was used for all the active ingredients mentioned in the comparative examples.

The drug release study details for individual active ingredients are enlisted below:

(i) Theophylline:

| | Acid stage: 0.1 N HCl (with or without alcohol) | Buffer stage: pH 6.8 |
|---|---|---|
| Dissolution volume (mL) | 750 | 900 |
| Speed (RPM) | 50 | 50 |

(ii) Metoprolol:

|  | Acid stage: 0.1 N HCl (with or without alcohol) | Buffer stage: pH 6.8 |
|---|---|---|
| Dissolution volume (mL) | 900 | 500 |
| Speed (RPM) | 50 | 50 |

(iii) Caffeine:

|  | Acid stage: 0.1 N HCl (with or without alcohol) | Buffer stage: pH 6.8 |
|---|---|---|
| Dissolution volume (mL) | 750 | 1000 |
| Speed (RPM) | 50 | 50 |

**(2) Results summary of comparative examples**

| Examples | C1 | C2 | C3 | C4 | C5 | C6 | C7 | C8 |
|---|---|---|---|---|---|---|---|---|
| Formulation details | Theophylline pellets coating with EUDRAGIT® FL 30 D-55 | Metoprolol pellets coated with mix of EUDRAGIT® NM 30D and sodium alginate | | | Caffeine pellets (16#-40#) coated with mix of EUDRAGIT® L 30D-55 and sodium alginate | | Bilayer coating on Caffeine pellets of inner layer sodium alginate and outer layer EUDRAGIT® L 30D-55 | Theophylline pellets coating with mix of EUDRAGIT® NM 30D+ EUDRAGIT® L 30 D-55 and sodium alginate |
| Coating dispersion Preparation time (min) | 60 | 120 | 120 | 120 | 120 | 130 | 160 | 75 |
| Ratio of polymer in coating | NA | NM:Sod alg = 70:30 | NM:Sod alg = 80:20 | NM:Sod alg = 90:10 | L:Sod alg = 40:60 | L:Sod alg = 66:33 | Inner layer: sodium alginate, Outer layer: EUDRAGIT® L 30D-55 | Physical mix: sodium alginate = 85:15 |
| Polymeric coat build up (% w/w) w.r.t core | 70% | 30% | 50% | 60% | 140% | 125% | 30% + 40% | 70% |
| Curing requirement | No | Yes | Yes | Yes | Yes | Yes | No | No |
| Enteric protection in 0.1 N HCl* (% release) | Yes (0.0) | No (96.1) | Yes (1.6) | Yes (0.3) | No (44.0) | No (20) | Yes (0.1) | Yes (8.3) |
| Extent of release in pH 6.8 buffer # (% release) | 100.0 | Not studied as enteric release failed | 97.1 | 64.0 | 85.0 | 77.0 | 100 | 89.4 |
| Enteric protection in 40% v/v alc 0.1N HCl ** (% release) | Yes (5.3) | No (62.6) | No (15.0) | No (19.3) | Yes (1.0) | No (18) | Yes (6.0) | No (15.3) |
| Extent of release in pH 6.8 buffet after alcoholic 0.1 N HCl preexposure ## (% release) | 31.5 | Not studied as enteric release failed | Not studied as enteric release failed | 77.7 | Not studied as enteric release failed | Not studied as enteric release failed | 100 | 93.0 |

EP 4 176 874 A1

| Examples | C1 | C2 | C3 | C4 | C5 | C6 | C7 | C8 |
|---|---|---|---|---|---|---|---|---|
| **Acceptance value (F2) after stability study (non-alcoholic media)** @ | Not done | Not done | Not done | Not done | Not done | Not done | 75.00 | 50.93 |
| **Acceptance value (F2) after stability study (alcoholic media)** @@ | Not done | Not done | Not done | Not done | Not done | Not done | 55.00 | 42.00 |
| **Comments** | Very high suppression in pH 6.8 buffer after preexposure to alcoholic acid | Alcohol resistance not observed. | Alcohol resistance not observed. | Alcohol resistance not observed. | Alcohol resistance not observed. | Alcohol resistance not observed. | Bilayer process requiring higher time for dispersion preparation and thus processing. | Alcohol resistance not observed. Difficult to process due to spray drying. Poor storage stability. |

*: Limit - Less than 10% release in 0.1N HCl, 2 hours

**: Limit - Less than 10% release in 2 hours in 0.1 N HCl containing 40% v/v ethanol.

#: Limit - Not less than 80% release in 3 hr in pH 6.8 buffer after preexposure to 0.1N HCl for 2 hrs.

##: Limit - Not less than 80% release in 3 hr in pH 6.8 buffer after 2 hour preexposure to 0.1N HCl containing 40% v/v ethanol.

@: Limit - F2 value of the dissolution profiles after 6 months storage of batches (at 40°C/ 75% RH) should be more than 50, when compared with the initial dissolution profile. Media: 0.1 N HCl (2 hrs) followed by pH 6.8 buffer (3 hrs).

@@: Limit - F2 value of the dissolution profiles after 6 months storage of batches (at 40°C/ 75% RH) should be more than 50, when compared with the initial dissolution profile. Media: 0.1 N HCl containing 40% v/v ethanol (2 hrs) followed by pH 6.8 buffer (3 hrs).

**D. Inventive Formula (I1, I2)**

**(1) Formula for coating of pellets with EUDRAGIT ® FL 30 D-55 and sodium alginate**

**I1: Substrate used: Theophylline pellets (18#-20#)**
**Batch size: 400g**

70% coating of pellets, 15% w/w solid content of dispersion

| Ingredients | Ratio | % on polymer | Solids | Qty required |
|---|---|---|---|---|
| | | | | |
| EUDRAGIT ® FL 30 D-55 | 85 | | 238.0 | 793.3 |
| Sodium alginate | 15 | | 42.0 | 42.0 |
| Talc | | 50% | 140.0 | 140.0 |
| Water | | | | 1824.6 |
| Total | | | 420.0 | 2800.0 |

**I2: Substrate used: Barrier coated Lansoprazole pellets (18#-20#)**

**Batch size: 400g**

70% coating of pellets, 15% w/w solid content of dispersion

| Ingredients | Ratio | % on polymer | Solids | Qty required |
|---|---|---|---|---|
| | | | | |
| EUDRAGIT ® FL 30 D-55 | 70 | | 196.0 | 653.3 |
| Sodium alginate | 30 | | 84.0 | 84.0 |
| Talc | | 50% | 140.0 | 140.0 |
| Water | | | | 1922.0 |
| Total | | | | 2799.3 |

**E. Process details for experiment I1, I2**

**(1) I1, I2: Preparation of coating solution for pellets coating**

I. To a part of water talc was homogenized for about for 15 min.
II. Some part of water was used to rinse the homogenizer and added to Step I.
III. Sodium alginate was dissolved in water under stirring for 30 min.
IV. Dispersion of EUDRAGIT® FL 30 D-55 was added to sodium alginate, slowly under stirring.
V. The dispersion of Step II was added to Step IV, under stirring.
VI. The final coating solution was passed through 40 # ASTM sieve (425 $\mu$m)

**(2) Coating of pellets**

The general process parameters are enlisted below:

| General Process Parameters in GPCG 1.1, bottom spray | | | |
|---|---|---|---|
| Parameters | Unit | Value (I1) | Value (I2) |
| **Equipment setup** | | | |
| Nozzle bore | Mm | 0.8 | 0.8 |
| Internal silicone tube diameter | Mm | 3 | 3 |
| Column Height | Mm | 16 | 15 |
| **Process parameter setup** | | | |
| Atomizing air pressure | Bar | 1.0 | 1.0 |
| Filter shaking Interval | Sec | 60 | 60 |
| Filter shaking | Sec | 05 | 05 |
| Air flow | $m^3/h$ | 50 - 60 | 50 - 65 |
| **Process data** | | | |
| Inlet Temperature | °C | 30 - 32 | 30 - 33 |
| Product Temperature | °C | 25 - 28 | 25 - 28 |
| Spray rate | (g/min) | 2-6 | 2-7 |

**Coated formulations were analyzed for drug release** in 0.1N HCl for 2 hours with and without presence of 40% v/v ethanol in it. Additionally, release in pH 6.8 phosphate buffer after preexposure to ethanolic or non-ethanolic acidic media was also carried out. Effect of curing on drug release in pH 6.8 (after preexposure to 0.1 N HCl for 2hours) was observed for inventive example I1 and storage stability on drug release in pH 6.8 (after preexposure to 0.1 N HCl for 2hours) was tested for I1 and I2. For all the dissolution/ drug release studies USP II apparatus was used for all the active ingredients mentioned in the inventive examples.

The drug release study details for individual active ingredients are enlisted below:

(i) Theophylline:

| | Acid stage: 0.1 N HCl (with or without alcohol) | Buffer stage: pH 6.8 |
|---|---|---|
| Dissolution volume (mL) | 750 | 900 |
| Speed (RPM) | 50 | 50 |

(ii) Lansoprazole:

| | Acid stage: 0.1 N HCl (with or without alcohol) | Buffer stage: pH 6.8 |
|---|---|---|
| Dissolution volume (mL) | 500 | 900 |
| Speed (RPM) | 75 | 75 |

**Results Summary of inventive example**

| Examples | I1 | I2 |
|---|---|---|
| Formulation details | Theophylline pellets coating with EUDRAGIT® FL 30 D-55 + sodium alginate | Lansoprazole pellets coating with EUDRAGIT® FL 30 D-55 + sodium alginate |
| Coating dispersion preparation time (min) | 45 | 45 |

18

(continued)

| Examples | I1 | I2 |
|---|---|---|
| Ratio of polymer in coating | FL: sodium alginate = 85:15 | FL: sodium alginate = 70:30 |
| Polymeric coat build up (% w/w) w.r.t core | 70% | 70% |
| Curing requirement | No | No |
| Enteric protection in 0.1 N HCl* (% release) | Yes (5.2) | Yes (2.9) |
| Extent of release in pH 6.8 buffer # (% release) | 94 | 100 |
| Enteric protection in 40% v/v alc 0.1 N HCl ** (% release) | Yes (8.2) | Yes (0.5) |
| Extent of release in pH 6.8 buffet after alcoholic 0.1 N HCl preexposure ## (% release) | 89 | 100 |
| Acceptance value (F2) after stability study (non-alcoholic media) @ | 91.60 | 89.00 |
| Acceptance value (F2) after stability study (alcoholic media) @@ | 81.26 | 76.00 |
| Comments | Acceptable alcohol resistance, release profiles and stability performance. Easy process. | Acceptable alcohol resistance, release profiles and stability performance. Easy process. |

*: Limit - Less than 10% release in 0.1N HCl, 2 hours

**: Limit - Less than 10% release in 2 hours in 0.1N HCl containing 40% v/v ethanol.

#: Limit - Not less than 80% release in 3 hr in pH 6.8 buffer after preexposure to 0.1 N HCl for 2 hrs.

##: Limit - Not less than 80% release in 3 hr in pH 6.8 buffer after 2 hour preexposure to 0.1 N HCl containing 40% v/v ethanol.

@: Limit - F2 value of the dissolution profiles after 6 months storage of batches (at 40°C/ 75% RH) should be more than 50, when compared with the initial dissolution profile. Media: 0.1 N HCl (2 hrs) followed by pH 6.8 buffer (3 hrs).

@@: Limit - F2 value of the dissolution profiles after 6 months storage of batches (at 40°C/ 75% RH) should be more than 50, when compared with the initial dissolution profile. Media: 0.1 N HCl containing 40% v/v ethanol (2 hrs) followed by pH 6.8 buffer (3 hrs).

The f2 Value/ f2 factor is a commonly used statistical measure to compare dissolution profiles of modified release dosage forms. The similarity factor f2, is a logarithmic reciprocal square root transformation of the sum of squared error and is a measurement of the similarity in the percent (%) of dissolution between the two curves.

$$f_2 = 50 \cdot \log \{[1 + (1/n)\Sigma_{t=1}^{n} (R_t - T_t)^2]^{-0.5} \cdot 100\}$$

The FDA has set a public standard of f2 value between 50-100 to indicate similarity between two dissolution profiles.

For the purpose of this invention, f2 value has been used to compare dissolution profiles behaviour and after storage stability studies to determine if the changes in dissolution profile, if any, are within acceptance limits.

**Claims**

1.  A gastric resistant pharmaceutical or nutraceutical composition, comprising a core, comprising a pharmaceutical or nutraceutical active ingredient and a single alcohol resistance conferring, gastric resistant coating layer onto the core, the coating layer comprising a mixture of 40 - 95 % by weight on the basis of the dry weight of the coating layer of an enteric core/shell polymer composition derived from an emulsion polymerisation process and of 5 - 60 % by weight on the basis of the dry weight of the coating layer of one or more salts of alginic acid, wherein the core of the core/shell polymer composition is formed by a water-insoluble copolymer and the shell of the core/shell polymer composition is formed by an anionic copolymer.

2. The composition of claim 1, wherein the composition further comprises one or more additional layers in order to achieve one or more of the functionalities, selected from modified release, odour masking, taste masking, improvement in optics, stabilization and moisture protection.

3. The composition of claim 1 or claim 2, wherein the anionic copolymer of the enteric core/shell polymer composition is an anionic (meth)acrylate copolymer comprising polymerized units of 40 to 60% by weight methacrylic acid, and 60 to 40% by weight ethyl acrylate.

4. The composition of any of claims 1 to 3, wherein the water-insoluble copolymer of the enteric core/shell polymer composition comprises (meth)acrylate copolymers.

5. The composition of claim 4, wherein the (meth)acrylate copolymers comprise polymerized units of 20 to 40 % by weight methyl (meth)acrylate and of 60 to 80 % by weight ethyl acrylate.

6. The composition of any of claims 1 to 5, wherein the one or more salts of alginic acid have a viscosity of 10 to 720 mPa·s in a 1 % aqueous solution.

7. The composition of any of claims 1 to 6, wherein the salt of alginic acid is sodium alginate.

8. The composition of any of the preceding claims, wherein the composition is comprised in a dosage form which is selected from tablets, pellets, granules, hard gelatine capsules and soft gelatine capsules.

9. A coating composition suitable for preparing the gastric resistant pharmaceutical or nutraceutical composition according to any of the preceding claims, comprising a mixture of 40 - 95 % by weight on the basis of the dry weight of the coating layer of an enteric core/shell polymer composition derived from an emulsion polymerisation process, wherein the core of the core/shell polymer composition is formed by a water-insoluble polymer or copolymer and the shell of the core/shell polymer composition is formed by an anionic copolymer and of 5 - 60 % by weight on the basis of the dry weight of the coating layer of one or more salts of alginic acid.

10. A process for producing the gastric resistant pharmaceutical or nutraceutical composition of any of claims 1 to 8 by forming the core comprising a pharmaceutical or nutraceutical active ingredient by applying the coating composition of claim 9 onto a core, comprising a pharmaceutical or nutraceutical active ingredient, using an aqueous system in a spray process or in a fluidized bed coating equipment coupled with bottom spray, tangential spray or a top spray assembly.

Europäisches
Patentamt

European
Patent Office

Office européen
des brevets

# EUROPEAN SEARCH REPORT

Application Number

EP 21 20 7041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | WO 2020/249505 A1 (EVONIK OPERATIONS GMBH [DE]) 17 December 2020 (2020-12-17) * page 11 - page 13; claim 1; examples I1-I20 * | 1-10 | INV. A61K31/138 A61K31/522 A61K9/28 A61K9/20 A61K31/4439 A61K9/16 |
| Y | US 9 844 511 B2 (NOLLENBERGER KATHRIN [DE]; SCHATTKA JAN HENDRIK [DE] ET AL.) 19 December 2017 (2017-12-19) * column 14; claim 1; table 1; compounds 3,4,7,8 * | 1-10 | |
| Y,D | WO 2014/032741 A1 (EVONIK INDUSTRIES AG [DE]) 6 March 2014 (2014-03-06) * page 21, paragraph 4 - page 23, paragraph 4; claim 1 * * page 109 - page 110; examples 21,22C * | 1-10 | |
| Y | WO 2016/193034 A1 (EVONIK RÖHM GMBH [DE]; JOSHI SHRADDHA SANJEEV [IN] ET AL.) 8 December 2016 (2016-12-08) * page 35 - page 39; claim 1; examples F8,F9,F19,F20 * | 1-10 | |

TECHNICAL FIELDS SEARCHED (IPC)

A61K

The present search report has been drawn up for all claims

| Place of search | Date of completion of the search | Examiner |
|---|---|---|
| Munich | 6 April 2022 | Cetinkaya, Murat |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
.......................................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 20 7041

This annex lists the patent family members relating to the patent documents cited in the above-mentioned European search report.
The members are as contained in the European Patent Office EDP file on
The European Patent Office is in no way liable for these particulars which are merely given for the purpose of information.

06-04-2022

| Patent document cited in search report | | Publication date | Patent family member(s) | | Publication date |
|---|---|---|---|---|---|
| WO 2020249505 | A1 | 17-12-2020 | BR | 112021025075 A2 | 01-02-2022 |
| | | | CA | 3141056 A1 | 17-12-2020 |
| | | | CN | 113950321 A | 18-01-2022 |
| | | | EP | 3982933 A1 | 20-04-2022 |
| | | | KR | 20220021488 A | 22-02-2022 |
| | | | WO | 2020249505 A1 | 17-12-2020 |
| US 9844511 | B2 | 19-12-2017 | AU | 2011370762 A1 | 14-11-2013 |
| | | | BR | 112013032095 A2 | 13-12-2016 |
| | | | CA | 2839689 A1 | 20-12-2012 |
| | | | CN | 103608001 A | 26-02-2014 |
| | | | EP | 2720682 A1 | 23-04-2014 |
| | | | ES | 2616006 T3 | 09-06-2017 |
| | | | HK | 1191572 A1 | 01-08-2014 |
| | | | HU | E033111 T2 | 28-11-2017 |
| | | | IL | 229166 A | 31-01-2018 |
| | | | JP | 5847303 B2 | 20-01-2016 |
| | | | JP | 2014518205 A | 28-07-2014 |
| | | | KR | 20140041543 A | 04-04-2014 |
| | | | MX | 353427 B | 12-01-2018 |
| | | | PL | 2720682 T3 | 31-05-2017 |
| | | | RU | 2014101227 A | 27-09-2015 |
| | | | US | 2014086997 A1 | 27-03-2014 |
| | | | WO | 2012171575 A1 | 20-12-2012 |
| WO 2014032741 | A1 | 06-03-2014 | AU | 2012388440 A1 | 22-01-2015 |
| | | | BR | 112015001371 A2 | 04-07-2017 |
| | | | CA | 2877502 A1 | 06-03-2014 |
| | | | CN | 104507460 A | 08-04-2015 |
| | | | EP | 2887925 A1 | 01-07-2015 |
| | | | ES | 2625017 T3 | 18-07-2017 |
| | | | HK | 1203841 A1 | 06-11-2015 |
| | | | HU | E031818 T2 | 28-08-2017 |
| | | | IL | 236313 A | 31-03-2020 |
| | | | JP | 6150896 B2 | 21-06-2017 |
| | | | JP | 2015528450 A | 28-09-2015 |
| | | | KR | 20150047493 A | 04-05-2015 |
| | | | MX | 355662 B | 25-04-2018 |
| | | | PL | 2887925 T3 | 31-07-2017 |
| | | | RU | 2015110825 A | 20-10-2016 |
| | | | US | 2015209298 A1 | 30-07-2015 |
| | | | WO | 2014032741 A1 | 06-03-2014 |
| WO 2016193034 | A1 | 08-12-2016 | BR | 112017025148 A2 | 07-08-2018 |
| | | | CA | 2986188 A1 | 08-12-2016 |
| | | | CN | 107708677 A | 16-02-2018 |

EPO FORM P0459

For more details about this annex : see Official Journal of the European Patent Office, No. 12/82

page 1 of 2

## ANNEX TO THE EUROPEAN SEARCH REPORT
## ON EUROPEAN PATENT APPLICATION NO.

EP 21 20 7041

This annex lists the patent family members relating to the patent documents cited in the above-mentioned European search report.
The members are as contained in the European Patent Office EDP file on
The European Patent Office is in no way liable for these particulars which are merely given for the purpose of information.

06-04-2022

| Patent document cited in search report | Publication date | Patent family member(s) | | Publication date |
|---|---|---|---|---|
| | | EP | 3302444 A1 | 11-04-2018 |
| | | ES | 2838816 T3 | 02-07-2021 |
| | | HK | 1244699 A1 | 17-08-2018 |
| | | HU | E052340 T2 | 28-05-2021 |
| | | JP | 6824197 B2 | 03-02-2021 |
| | | JP | 2018518478 A | 12-07-2018 |
| | | KR | 20180015245 A | 12-02-2018 |
| | | SI | 3302444 T1 | 29-01-2021 |
| | | US | 2018140556 A1 | 24-05-2018 |
| | | US | 2021205230 A1 | 08-07-2021 |
| | | WO | 2016193034 A1 | 08-12-2016 |

EPO FORM P0459

For more details about this annex : see Official Journal of the European Patent Office, No. 12/82

page 2 of 2

**EP 4 176 874 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2012171575 A1
- WO 2012171576 A1
- US 20120093926 A1
- WO 2012022498 A1
- WO 2020249505 A1
- WO 2020225122 A1
- WO 2014032741 A1
- WO 2014032742 A1
- WO 2015121189 A1
- WO 0168767 A1

### Non-patent literature cited in the description

- methods of analysis. European Pharmacopeia. vol. 2.2.8-2.2.10, 27ff